# EUROPEAN PATENT APPLICATION

(11) **EP 4 047 370 A1**
(43) Date of publication of application: **24.08.2022**
(21) Application number: 22157860.2
(22) Date of filing: 22.02.2022
(51) Int. Cl.: G01N 33/569, G01N 33/68

(54) **METHOD FOR ASSISTING DETERMINATION OF EXACERBATION RISK OF COVID-19, USE OF REAGENT KIT, APPARATUS FOR ACQUIRING INFORMATION ON EXACERBATION RISK OF COVID-19**

(30) Priority: 22.02.2021 JP 2021026649
(71) Applicant: National Center for Global Health and Medicine, Tokyo 162-8655 (JP); National Cancer Center, Tokyo 104-0045 (JP); SYSMEX CORPORATION, Kobe-shi Hyogo 651-0073 (JP)
(72) Inventor: MITSUYA, Hiroaki, Tokyo, 1628655 (JP); MAEDA, Kenji, Tokyo, 1628655 (JP); HAMADA, Akinobu, Tokyo, 1040045 (JP); NODA, Kenta, Kobe-shi, Hyogo, 651-0073 (JP); YAMASHITA, Kazuto, Kobe-shi, Hyogo, 651-0073 (JP); ATARASHI, Yusuke, Kobe-shi, Hyogo, 651-0073 (JP); IDE, Nobuyuki, Kobe-shi, Hyogo, 651-0073 (JP)
(74) Representative: De Clercq & Partners

(57) **Abstract**

Disclosed is a method for acquiring information on exacerbation risk of COVID-19, comprising measuring IgM antibody against S antigen of SARS-CoV-2 contained in a specimen collected from a subject infected with SARS-CoV-2 or a subject suspected of suffering from COVID-19, wherein a value obtained by the measurement of IgM antibody serves as an index of exacerbation risk of COVID-19 of the subject.

## Description

### FIELD OF THE INVENTION

The present invention relates to a method for assisting determination of exacerbation risk of COVID-19. The present invention relates to use of a reagent kit for the method. The present invention relates to an apparatus for acquiring information on exacerbation risk of COVID-19.

### BACKGROUND

COVID-19 patients are often mild and do not require hospitalization, but some patients become severe and need to be hospitalized for treatment. When COVID-19 becomes the most severe, it is necessary to preferentially perform advanced treatment such as artificial respiration management on the patient. Therefore, there is a high need for means for predicting exacerbation of COVID-19. Han H. et al., (Profiling serum cytokines in COVID-19 patients reveals IL-6 and IL-10 are disease severity predictors, Emerg Microbes Infect., 2020, vol. 9, pp. 1123-1130) describes that when COVID-19 patients are classified into mild, moderate and severe, and various cytokines in the serum of each patient are measured, the measured values of IL (Interleukin)-6 and IL-10 of the severe patient group are significantly higher than those of the mild and moderate patient groups. From this result, Han H. et al., (above) describes that IL-6 and IL-10 can be predictive markers of exacerbation of COVID-19.

### SUMMARY OF THE INVENTION

The publication by Han H. et al. (above) focuses on inflammatory cytokines produced in patients by SARS-CoV-2 infection as biomarkers associated with exacerbation of COVID-19. On the other hand, it is generally known that when a patient is infected with a virus including SARS-CoV-2, an antibody against a protein (antigen) of the virus is generated in the body of the patient. The present inventors focused on an antibody against SARS-CoV-2 antigen, which occurs in a patient due to SARS-CoV-2 infection, as a biomarker. An object is to provide a new means for acquiring information on exacerbation risk of COVID-19 using the measured value of antibody against SARS-CoV-2 antigen in a specimen as an index. That is, an object is to provide a method for acquiring information on exacerbation risk of COVID-19, a method for monitoring IgM antibody against S antigen of SARS-CoV-2, a method for assisting determination of exacerbation risk of COVID-19, a reagent kit, an apparatus for acquiring information on exacerbation risk of COVID-19, and a computer program for acquiring information on exacerbation risk of COVID-19.

Provided is a method for assisting determination of exacerbation risk of COVID-19, including measuring IgM antibody against S antigen of SARS-CoV-2 contained in a specimen collected from a subject infected with SARS-CoV-2 or a subject suspected of suffering from COVID-19, and determining the exacerbation risk of COVID-19 of the subject, based on a value obtained by the measurement of IgM antibody.

Provided is a method for acquiring information on exacerbation risk of COVID-19, including measuring IgM antibody against S antigen of SARS-CoV-2 contained in a specimen collected from a subject infected with SARS-CoV-2 or a subject suspected of suffering from COVID-19, in which a value obtained by the measurement of IgM antibody serves as an index of exacerbation risk of COVID-19 of the subject.

Provided is a method for monitoring IgM antibody against S antigen of SARS-CoV-2, using a first specimen collected at a first time point from a subject infected with SARS-CoV-2 or a subject suspected of suffering from COVID-19 and a second specimen collected at a second time point from the subject, comprising measuring IgM antibody against S antigen of SARS-CoV-2 contained in each of the first and second specimens, wherein values obtained by the measurements of IgM antibody serve as indices of exacerbation risk of COVID-19 of the subject.

Provided is use of a reagent kit in any of the above methods, including a reagent containing a substance capable of specifically binding to IgM antibody against S antigen of SARS-CoV-2.

Provided is an apparatus for acquiring information on exacerbation risk of COVID-19, including a computer containing a processor and a memory under control of the processor, in which the memory is recorded with a computer program for executing on the computer the steps of acquiring a value obtained by measurement of IgM antibody against S antigen of SARS-CoV-2 contained in a specimen collected from a subject infected with SARS-CoV-2 or a subject suspected of suffering from COVID-19, and outputting the value, and wherein the value serves as an index of exacerbation risk of COVID-19 of the subject.

According to the invention, it is possible to determine the exacerbation risk of COVID-19 of the subject.

### BRIEF DESCRIPTION OF THE DRAWINGS

Fig. 1A is a schematic diagram showing an example of the reagent kit of the present invention.
Fig. 1B is a schematic diagram showing an example of the reagent kit of the present invention.
Fig. 1C is a schematic diagram showing an example of the reagent kit of the present invention.
Fig. 2 is a schematic diagram showing an example of the acquisition apparatus of the present invention.
Fig. 3 is a block diagram showing a hardware configuration of the acquisition apparatus of the present invention.
Fig. 4A is a flowchart showing a processing procedure by the acquisition apparatus of the present invention.
Fig. 4B is a flowchart showing a processing procedure by the acquisition apparatus of the present invention.
Fig. 4C is a flowchart showing a processing procedure by the acquisition apparatus of the present invention.

### DETAILED DESCRIPTION OF THE PREFERRED EMBODIMENTS

In the method for acquiring information on exacerbation risk of COVID-19 of the present invention (hereinafter also referred to as "acquisition method"), IgM antibody against S antigen of SARS-CoV-2 (hereinafter also referred to as "S-IgM") contained in a specimen collected from a subject infected with SARS-CoV-2 or a subject suspected of suffering from COVID-19 is measured. In the method of the present invention, SARS-CoV-2 includes not only a virus whose genome sequence was first determined (Severe acute respiratory syndrome coronavirus 2 isolate Wuhan-Hu-1, GenBank accession number: MN908947.3) but also subspecies thereof.

Examples of the subject in the method of the present invention include a subject infected with SARS-CoV-2 and a subject suspected of suffering from COVID-19. The subject infected with SARS-CoV-2 refers to a person whose infection has been confirmed by detection of SARS-CoV-2 by a known test such as a PCR test or an antigen test. The subject infected with SARS-CoV-2 includes a person who developed COVID-19 (COVID-19 patient) and a person who is asymptomatic. When the subject is a COVID-19 patient, the severity thereof is preferably mild, moderate without respiratory failure, or moderate with respiratory failure. The mild means, for example, a condition in which oxygen saturation (SpO₂) is 96% or more, there is no respiratory symptom, and there is only cough and no shortness of breath. The moderate without respiratory failure refers to, for example, a condition in which SpO₂ is higher than 93% and less than 96%, shortness of breath is present, and pneumonia is observed. The moderate with respiratory failure refers to, for example, a condition in which SpO₂ is 93% or less and oxygen administration is necessary.

The subject suspected of suffering from COVID-19 includes a person who has symptoms found in COVID-19 but has not been tested for SARS-CoV-2, a person who had contact with a SARS-CoV-2 infected person or a COVID-19 patient, and a person suspected of having contact. The subject suspected of suffering from COVID-19 can also be said to be a person suspected of being infected with SARS-CoV-2. Symptoms seen in COVID-19 include cold symptoms such as fever, cough, runny nose and sore throat, and/or breathlessness, shortness of breath on exertion, abnormal taste and smell, and the like. Contact with an infected person or a patient refers to, for example, an act such as talking with an infected person or a patient within a distance of 1 m, staying in a closed space where an infected person or a patient is present, and being splashed with saliva, coughing or the like of an infected person or a patient.

The specimen is not particularly limited as long as it is a sample collected from a subject and can contain S-IgM antibody. Examples of such sample include blood samples, lymph fluid, cerebrospinal fluid, saliva, nasopharyngeal swab, sputum, bronchoalveolar lavage fluid, urine, stool, and the like. Examples of the blood sample include blood (whole blood) collected from a subject and plasma or serum prepared from the blood. In the present invention, whole blood, plasma and serum are preferred, and plasma and serum are particularly preferred.

When insoluble contaminants such as cells are contained in the specimen, for example, impurities may be removed from the specimen by a known means such as centrifugal separation and filtration. The specimen may be diluted with an appropriate aqueous medium as necessary. The aqueous medium is not particularly limited as long as it does not interfere with the measurement of biomarker described later. Examples of the aqueous medium include water, physiological saline, a buffer solution, and the like. The buffer solution is not particularly limited as long as it has a buffering effect at a pH near neutrality (for example, a pH of 6 or more and 8 or less). Examples of the buffer solution include Good buffers such as HEPES, MES, and PIPES, phosphate buffered saline (PBS), tris hydrochloric acid buffer, tris buffered saline (TBS), and the like.

In the acquisition method of the present invention, S-IgM is measured as a biomarker. S-IgM is an IgM antibody against a spike protein of SARS-CoV-2 that occurs in the body of a subject due to infection with SARS-CoV-2. The spike protein of SARS-CoV-2 is also called S antigen. The S antigen of SARS-CoV-2 is known per se, and an amino acid sequence thereof can be acquired from known databases such as NCBI (National Center for Biotechnology Information).

As used herein, the phrase "measuring IgM antibody against S antigen of SARS-CoV-2" includes acquiring a value that reflects the amount or concentration of S-IgM, and determining a value of the amount or concentration of S-IgM. The phrase "value that reflects the amount or concentration of S-IgM" is a value depending on the type of the labeling substance described later, and it can be acquired by a measuring device according to the type of the labeling substance. Examples of such value include a measured value of emission intensity, a measured value of fluorescence intensity, a measured value of radiation intensity, a measured value of optical density, and the like. The phrase "value of the amount or concentration of S-IgM" can be determined based on the value that reflects the amount or concentration of S-IgM and the measurement result of a calibrator. The calibrator is a kind of control sample, and is a sample for quantification of a test substance containing a test substance or a standard substance corresponding thereto at a known concentration.

In the present invention, for example, a commercially available SARS-CoV-2 IgM positive specimen (plasma or serum) can be used as a calibrator.

In the present invention, a value obtained by measurement of S-IgM (hereinafter also referred to as "measured value of S-IgM") can be a value that reflects the amount or concentration of S-IgM in the specimen. The measured value of S-IgM may be a value of the amount or concentration of S-IgM in the specimen, determined based on the measurement result of the calibrator.

A means for measuring S-IgM is not particularly limited, and can be appropriately selected from known measurement methods. In the present invention, a method including capturing S-IgM using a substance capable of specifically binding to S-IgM is preferred. S-IgM contained in the specimen can be measured by detecting S-IgM captured by such a substance by a known method.

Examples of the substance capable of specifically binding to S-IgM include S antigen of SARS-CoV-2 (hereinafter, also simply referred to as "S antigen"), an antibody, an aptamer, and the like. Among them, S antigen is preferred, and in particular, an S1 subunit of S antigen is preferred. The S antigen may be a naturally occurring protein or a recombinant protein. Natural S antigen can be isolated, for example, from a SARS-CoV-2 positive specimen by a conventional method. Recombinant S antigen can be obtained by known methods such as DNA recombination technology and other molecular biological techniques. First, a polynucleotide encoding S antigen is incorporated into a known protein expression vector to obtain an S antigen expression vector. By transforming or transfecting the obtained S antigen expression vector into an appropriate host cell, recombinant S antigen can be obtained. Base sequence of the polynucleotide encoding S antigen is known per se, and can be obtained from a known database such as NCBI. The type of the protein expression vector is not particularly limited, and it may be a vector for mammalian cells or a vector for E. coli.

The method for measuring S-IgM using S antigen is not particularly limited, and can be appropriately selected from known immunological measurement methods such as enzyme-linked immunosorbent assay (ELISA), enzyme immunoassay, immunoturbidimetry, immunonephelometry, and latex agglutination. In the present invention, the ELISA is preferred. The type of the ELISA may be any of a sandwich method, a competitive method, a direct method, an indirect method and the like, and the sandwich method is particularly preferred. As an example, the case of measuring S-IgM in the specimen by sandwich ELISA will be described below.

Measurement of S-IgM by sandwich ELISA using S antigen includes a process of forming a complex of S antigen and S-IgM and a process of detecting the complex. In the process of forming a complex, a complex containing S-IgM, S antigen, and an antibody for detecting S-IgM (hereinafter also referred to as "S-IgM detection antibody") is formed on a solid phase. In the sandwich ELISA method, the S antigen functions as a substance for capturing S-IgM. When the specimen contains S-IgM, a complex containing S-IgM, S antigen and S-IgM detection antibody can be formed by mixing the specimen, S antigen, and S-IgM detection antibody. The complex can be formed on the solid phase by contacting a solution containing the complex with a solid phase on which the S antigen can be immobilized. Alternatively, a solid phase on which the S antigen has been previously immobilized may be used. That is, the complex can be formed on the solid phase by contacting the specimen, the solid phase on which the S antigen has been immobilized, and the S-IgM detection antibody with each other. In another embodiment, in the process of forming a complex, a complex containing S-IgM, an S antigen for detecting S-IgM, and an antibody for capturing S-IgM (hereinafter also referred to as "S-IgM capture antibody") is formed on the solid phase.

The S-IgM detection antibody and the S-IgM capture antibody are not particularly limited as long as they are antibodies capable of specifically binding to S-IgM or human IgM. As used herein, the term "antibody" includes full-length antibodies and fragments thereof. Examples of the fragment of the antibody include reduced IgG (rIgG), Fab, Fab', F(ab')2, Fv, single chain antibody (scFv), diabody, triabody, and the like. The antibody may be either a monoclonal antibody or a polyclonal antibody. The antibody capable of specifically binding to S-IgM may be obtained, for example, by preparing a hybridoma producing the antibody by the method described in Kohler G. and Milstein C., Nature, vol. 256, pp. 495-497, 1975. Antibodies capable of specifically binding to human IgM are known per se and commercially available.

The solid phase may be an insoluble carrier capable of immobilizing the S antigen or the S-IgM capture antibody. The mode of immobilization of the S antigen or the S-IgM capture antibody on the solid phase is not particularly limited. For example, the S antigen or the S-IgM capture antibody and the solid phase may be bound directly, or the S antigen or the S-IgM capture antibody and the solid phase may be indirectly bound via another substance. Examples of the direct binding include physical adsorption and covalent bond by a crosslinking agent, and the like. As the indirect binding, for example, the S antigen or the S-IgM capture antibody can be immobilized on the solid phase using a combination of substances interposed between the S antigen or the S-IgM capture antibody and the solid phase. Examples of the combination of substances include combinations of any of biotin and its analogs and any of biotin-binding sites, a hapten and an anti-hapten antibody and the like. The biotin and its analogs include biotin and biotin analogs such as desthiobiotin and oxybiotin. The biotin-binding sites include avidin and avidin analogs such as streptavidin and tamavidin (registered trademark). Examples of the combination of a hapten and an anti-hapten antibody include a combination of a compound having a 2,4-dinitrophenyl (DNP) group and an anti-DNP antibody. For example, by using an S antigen or an S-IgM capture antibody previously modified with biotin or its analog (or a compound having a DNP group) and a solid phase to which a biotin-binding site (or anti-DNP antibody) is previously bound, the S antigen or the S-IgM capture antibody can be immobilized on the solid phase through binding between the biotin or its analog and the biotin-binding site (or binding between the DNP group and the anti-DNP antibody).

The material of the solid phase is not particularly limited. For example, the material can be selected from organic polymer compounds, inorganic compounds, biopolymers, and the like. Examples of the organic polymer compound include latex, polystyrene, polypropylene, and the like. Examples of the inorganic compound include magnetic bodies (iron oxide, chromium oxide, ferrite, and the like), silica, alumina, glass, and the like. Examples of the biopolymer include insoluble agarose, insoluble dextran, gelatin, cellulose, and the like. Two or more of these may be used in combination. The shape of the solid phase is not particularly limited, and examples thereof include a particle, a membrane, a microplate, a microtube, a test tube, and the like. Among them, a particle is preferred, and a magnetic particle is particularly preferred.

In the methods of the present invention, B/F (Bound/Free) separation for removing an unreacted free component not forming a complex may be performed between the process of forming the complex and the process of detecting the complex. The unreacted free component refers to a component not constituting a complex. Examples thereof include S antigen not bound to S-IgM, detection antibodies, and the like. The means of B/F separation is not particularly limited, and when the solid phase is a particle, B/F separation can be performed by recovering only the solid phase capturing the complex by centrifugation. When the solid phase is a container such as a microplate or a microtube, B/F separation can be performed by removing a liquid containing an unreacted free component. When the solid phase is a magnetic particle, B/F separation can be performed by aspirating and removing a liquid containing an unreacted free component by a nozzle while magnetically constraining the magnetic particle with a magnet, which is preferable from the viewpoint of automation. After removing the unreacted free component, the solid phase capturing the complex may be washed with a suitable aqueous medium such as PBS.

In the process of detecting the complex, the measured value of S-IgM can be acquired by detecting the complex formed on the solid phase by a method known in the art. For example, when an antibody labeled with a labeling substance is used as S-IgM detection antibody, the measured value of S-IgM can be acquired by detecting a signal generated by the labeling substance. Alternatively, also when a labeled secondary antibody against the S-IgM detection antibody is used, the measured value of S-IgM can be acquired in the same manner.

As used herein, the phrase "detecting a signal" includes qualitatively detecting the presence or absence of a signal, quantifying a signal intensity, and semi-quantitatively detecting the signal intensity. Semi-quantitative detection means to show the signal intensity in stages like "no signal generated", "weak", "medium", "strong", and the like. In the methods of the present invention, it is preferable to detect the signal intensity quantitatively or semi-quantitatively, and it is particularly preferable to detect the signal intensity quantitatively.

The labeling substance is not particularly limited. For example, the labeling substance may be a substance which itself generates a signal (hereinafter also referred to as "signal generating substance") or a substance which catalyzes the reaction of other substances to generate a signal. Examples of the signal generating substance include fluorescent substances, radioactive isotopes, and the like. Examples of the substance that catalyzes the reaction of other substances to generate a detectable signal include enzymes. Examples of the enzymes include alkaline phosphatase (ALP), peroxidase, β-galactosidase, luciferase, and the like. Examples of the fluorescent substances include fluorescent dyes such as fluorescein isothiocyanate (FITC), rhodamine and Alexa Fluor (registered trademark), fluorescent proteins such as GFP, and the like. Examples of the radioactive isotopes include ¹²⁵I, ¹⁴C, ³²P, and the like. As the labeling substance, an enzyme is preferred, and ALP is particularly preferred.

Methods for detecting a signal are known per se in the art. In the present invention, a measurement method according to the type of signal derived from the labeling substance may be appropriately selected. For example, when the labeling substance is an enzyme, signals such as light and color generated by reacting a substrate for the enzyme can be measured by using a known apparatus such as a spectrophotometer.

The substrate of the enzyme can be appropriately selected from known substrates according to the type of the enzyme. For example, when alkaline phosphatase is used as the enzyme, examples of the substrate include chemiluminescent substrates such as CDP-Star (registered trademark) (disodium 4-chloro-3-(methoxyspiro[1,2-dioxetane-3,2'-(5'-chloro)tricyclo[3.3.1.13,7]decan]-4-yl)phenyl phosphate) and CSPD (registered trademark) (disodium 3-(4-methoxyspiro[1,2-dioxetane-3,2-(5'-chloro)tricyclo[3.3.1.13,7]decan]-4-yl)phenyl phosphate), and chromogenic substrates such as 5-bromo-4-chloro-3-indolyl phosphate (BCIP), disodium 5-bromo-6-chloro-indolyl phosphate and p-nitrophenyl phosphate. Also, when peroxidase is used as the enzyme, examples of the substrate include chemiluminescent substrates such as luminol and derivatives thereof, and chromogenic substrates such as 2,2'-azinobis(3-ethylbenzothiazoline-6-ammonium sulfonate) (ABTS), 1,2-phenylenediamine (OPD) and 3,3',5,5'-tetramethylbenzidine (TMB).

When the labeling substance is a radioactive isotope, radiation as a signal can be measured using a known apparatus such as a scintillation counter. Also, when the labeling substance is a fluorescent substance, fluorescence as a signal can be measured using a known apparatus such as a fluorescence microplate reader. The excitation wavelength and the fluorescence wavelength can be appropriately determined according to the type of fluorescent substance used.

The detection result of the signal can be used as the measurement result of S-IgM. For example, when quantitatively detecting the signal intensity, a measured value of signal intensity itself or a value acquired from the measured value can be used as the measured value of S-IgM. Examples of the value acquired from the measured value of the signal intensity include a value obtained by subtracting the measured value of a negative control sample or the background value from the measured value, and the like. The negative control sample can be appropriately selected, and examples thereof include a buffer solution containing no S-IgM, a specimen obtained from a healthy person who has not been infected with SARS-CoV-2, and the like.

In the methods of the present invention, it is preferable to measure S-IgM contained in the specimen by sandwich ELISA using an S antigen immobilized on a magnetic particle and an enzyme-labeled detection antibody. Measurement may be carried out using a commercially available measuring device such as HISCL (registered trademark) series (manufactured by Sysmex Corporation).

As shown in examples described later, serum S-IgM concentration in a patient group in which COVID-19 became severe was significantly higher than that in a patient group in which COVID-19 did not become severe. As described above, the measurement result of S-IgM can be used as an index of exacerbation risk of COVID-19 of a subject. In the present invention, the exacerbation risk of COVID-19 of a subject is a possibility of exacerbation of COVID-19 of the subject after a lapse of a predetermined period (for example, 1 day to 1 month) from a date when the specimen was collected from the subject. In one embodiment, exacerbation of COVID-19 refers to a condition in which intensive care management including artificial respiration management is necessary, or a condition in which introduction of extracorporeal membrane oxygenation (ECMO) is necessary.

In the methods of the present invention, by comparing the measured value of S-IgM with a predetermined threshold value, the measured value of S-IgM may be used as an index of exacerbation risk of COVID-19 of a subject. In one embodiment, when the measured value of S-IgM is greater than or equal to the predetermined threshold value, it is suggested that the exacerbation risk of COVID-19 of the subject is high. In a further embodiment, when the measured value of S-IgM is less than the predetermined threshold value, it is suggested that the exacerbation risk of COVID-19 of the subject is low.

In a further embodiment, S-IgM may be combined with other biomarker. Examples of other biomarker include IL-4. IL-4 is one of Th2 cytokines and is known to be involved in class switching from IgM to IgG. Amino acid sequence of IL-4 can be obtained from a known database such as NCBI. One embodiment is a method for acquiring information on exacerbation risk of COVID-19, including measuring a biomarker contained in a specimen collected from a subject infected with SARS-CoV-2, in which a value obtained by the measurement of biomarker serves as an index of exacerbation risk of COVID-19 of the subject, and the biomarker includes IgM antibody against S antigen of SARS-CoV-2 and IL-4.

In another embodiment, IL-4 may be measured instead of S-IgM. In this case, the measured value of IL-4 serves as an index of exacerbation risk of COVID-19 of a subject. One embodiment is a method for acquiring information on exacerbation risk of COVID-19, including measuring IL-4 contained in a specimen collected from a subject infected with SARS-CoV-2 or a subject suspected of suffering from COVID-19, in which a value obtained by the measurement of IL-4 serves as an index of exacerbation risk of COVID-19 of the subject.

As used herein, the phrase "measuring IL-4" includes acquiring a value that reflects the amount or concentration of IL-4, and determining a value of the amount or concentration of IL-4. The phrase "value that reflects the amount or concentration of IL-4" is a value depending on the type of the labeling substance, and it can be acquired by a measuring device according to the type of the labeling substance. The phrase "value of the amount or concentration of IL-4" can be determined based on the value that reflects the amount or concentration of IL-4 and the measurement result of a calibrator. In the methods of the present invention, for example, a recombinant protein of IL-4 can be used as a calibrator.

In the methods of the present invention, a value obtained by measurement of IL-4 (hereinafter also referred to as "measured value of IL-4") can be a value that reflects the amount or concentration of IL-4 in the specimen. The measured value of IL-4 may be a value of the amount or concentration of IL-4 in the specimen, determined based on the measurement result of the calibrator.

A means for measuring IL-4 is not particularly limited, and can be appropriately selected from known measurement methods. In the present invention, a method including capturing IL-4 using a substance capable of specifically binding to IL-4 is preferred. IL-4 contained in the specimen can be measured by detecting IL-4 captured by such a substance by a known method. Examples of the substance capable of specifically binding to IL-4 include an antibody, an aptamer, and the like. The antibody is particularly preferred among them. Antibodies specifically binding to IL-4 are known per se and commercially available. A method for measuring IL-4 using an antibody is not particularly limited and can be appropriately selected from known immunological measurement methods. In the present invention, the ELISA is preferred.

As an example, measurement of IL-4 by sandwich ELISA will be described below. This measurement includes a process of forming a complex of an antibody and IL-4 and a process of detecting the complex. In the process of forming a complex, a complex containing IL-4, an antibody for capturing IL-4 (hereinafter also referred to as "capture antibody"), and an antibody for detecting IL-4 (hereinafter also referred to as "IL-4 detection antibody") is formed on a solid phase. Details of the solid phase are as described above. When the specimen contains IL-4, a complex containing IL-4, a capture antibody, and IL-4 detection antibody can be formed by mixing the specimen, the capture antibody, and the IL-4 detection antibody. The complex can be formed on the solid phase by contacting a solution containing the complex with a solid phase on which the capture antibody can be immobilized. Alternatively, a solid phase on which the capture antibody has been preliminarily immobilized may be used. That is, a solid phase on which the capture antibody has been immobilized, the specimen, and the IL-4 detection antibody are contacted with each other, whereby the complex can be formed on the solid phase. When both the capture antibody and the IL-4 detection antibody are monoclonal antibodies, it is preferable that the epitopes be different from each other.

Details of the process of detecting the complex containing IL-4, the capture antibody and the IL-4 detection antibody are the same as those described for the measurement of S-IgM. In a preferred embodiment, IL-4 detection antibody labeled with a labeling substance is used, and the measured value of IL-4 is acquired by detecting a signal generated by the labeling substance. Alternatively, also when a labeled secondary antibody against the IL-4 detection antibody is used, the measured value of IL-4 can be acquired in the same manner. The detection result of the signal can be used as the measurement result of IL-4. For example, when quantitatively detecting the signal intensity, a measured value of signal intensity itself or a value acquired from the measured value can be used as the measured value of IL-4.

In the methods of the present invention, it is preferable to measure IL-4 contained in the specimen by sandwich ELISA using a capture antibody immobilized on a magnetic particle and an enzyme-labeled detection antibody.

Similar to S-IgM, serum IL-4 concentration in the patient group with severe COVID-19 was significantly higher than that in the patient group in which COVID-19 did not become severe. Therefore, the measurement result of IL-4 can also be used as an index of exacerbation risk of COVID-19 of a subject.

In a further embodiment, by comparing the measured values of S-IgM and IL-4 with predetermined threshold values corresponding thereto respectively, the measured values of S-IgM and IL-4 may be used as an index of exacerbation risk of COVID-19 of a subject. Hereinafter, the predetermined threshold value corresponding to S-IgM is referred to as a "first threshold value", and the predetermined threshold value corresponding to IL-4 is referred to as a "second threshold value". In one embodiment, when the measured value of S-IgM is greater than or equal to the first threshold value, or the measured value of IL-4 is greater than or equal to the second threshold value, it is suggested that the exacerbation risk of COVID-19 of the subject is high. In one embodiment, when the measured value of S-IgM is less than the first threshold value, and the measured value of IL-4 is less than the second threshold value, it is suggested that the exacerbation risk of COVID-19 of the subject is low.

In a further embodiment, the acquisition method includes measuring S-IgM and IL-4 contained in a specimen collected from a subject, and classifies the exacerbation risk of COVID-19 of the subject into three stages by the measured values of S-IgM and IL-4. Specifically, the acquisition method is as follows:
- when the measured value of S-IgM is greater than or equal to the first threshold value and the measured value of IL-4 is greater than or equal to the second threshold value, it is suggested that the exacerbation risk of COVID-19 of the subject is high;
- when the measured value of S-IgM is greater than or equal to the first threshold value or the measured value of IL-4 is greater than or equal to the second threshold value, it is suggested that the exacerbation risk of COVID-19 of the subject is moderate; and
- when the measured value of S-IgM is less than the first threshold value and the measured value of IL-4 is less than the second threshold value, it is suggested that the exacerbation risk of COVID-19 of the subject is low.

In another embodiment, by comparing the measured value of IL-4 with the second threshold value, the measured value of IL-4 may be used as an index of exacerbation risk of COVID-19 of a subject. In one embodiment, when the measured value of IL-4 is greater than or equal to the second threshold value, it is suggested that the exacerbation risk of COVID-19 of the subject is high. In one embodiment, when the measured value of IL-4 is less than the second threshold value, it is suggested that the exacerbation risk of COVID-19 of the subject is low.

The threshold values corresponding to S-IgM and IL-4 respectively are not particularly limited and can be set as appropriate. For example, specimens are collected from a plurality of SARS-CoV-2 infected persons or COVID-19 patients, and S-IgM and IL-4 in the specimens are measured. After a predetermined period (for example, 2 weeks) has passed since the specimens were collected, whether or not COVID-19 has become severe is confirmed. Data of the measured values of S-IgM and IL-4 is classified into data of a group of severely ill patients and data of a group of non-severely ill patients. Then, for each of S-IgM and IL-4, a value that can most accurately distinguish between the group of severely ill patients and the group of non-severely ill patients is determined, and the value is set as a threshold value. In setting the threshold value, it is possible to consider sensitivity, specificity, positive predictive value, negative predictive value, and the like.

In one embodiment, the predetermined threshold value corresponding to S-IgM is set in a range of, for example, 35 AU/mL or more and 42 AU/mL or less, preferably 37 AU/mL or more and 41 AU/mL or less, and more preferably 38 AU/mL or more and 41 AU/mL or less. Since it is considered that IL-4 is hardly detected in a subject with low exacerbation risk, the predetermined threshold value corresponding to IL-4 can be, for example, a detection limit of measurement kit. Specifically, the predetermined threshold value corresponding to IL-4 is set in a range of 0.1 pg/mL or more and 2 pg/mL or less, preferably 0.5 pg/mL or more and 1.5 pg/mL or less, and more preferably 0.8 pg/mL or more and 1.2 pg/mL or less.

A healthcare professional such as a doctor may combine the suggestion from the measured values of S-IgM and/or IL-4 with other information to determine COVID-19 of a subject. The "other information" includes findings on X-ray or CT images of the lungs and other medical findings.

In the methods of the present invention, a temporal change of the measured values of S-IgM and/or IL-4 may be obtained. In this case, the temporal change of the measured values of S-IgM and/or IL-4 serves as an index of exacerbation risk of COVID-19 of a subject. The temporal change of the measured value is not particularly limited as long as it is information showing transition of the measured values of S-IgM and/or IL-4 in the specimens collected from the same subject a plurality of times periodically or irregularly. Examples of such temporal change include values calculated from a plurality of measured values (for example, the difference, ratio, etc. of the measured values of two specimens collected from the subject at any two time points), records of the measured values (for example, a table of measured values, a graph plotting measured values, etc.), and the like.

According to the present invention, when the exacerbation risk of COVID-19 of the subject is suggested to be high by the measured values of S-IgM and/or IL-4, it is possible to perform medical intervention for severe COVID-19. Examples of the medical intervention include drug administration, surgery, immunotherapy, gene therapy, oxygenation procedures, heart-lung machine procedures, and the like. The drug can be appropriately selected from known therapeutic drugs for COVID-19 or candidate medicines therefor. Examples thereof include drugs having antiviral action, drugs for reducing inflammation, ACE inhibitors, and the like. Specific examples of the drug include favipiravir, lopinavir, ritonavir, nafamostat, camostat, remdesivir, ribavirin, ivermectin, ciclesonide, chloroquine, hydroxychloroquine, interferon, tocilizumab, sarilumab, tofasitinib, baricitinib, ruxolitinib, acalabrutinib, ravulizumab, eritoran, ibudilast, HLCM051, LY3127804, and the like. Also, in another embodiment, the drug is a vaccine. Examples of the vaccine include viral vector vaccines, mRNA vaccines, DNA vaccines, recombinant protein vaccines, VLP vaccines, inactivated vaccines, and the like.

One further related aspect provided herein is a method for assisting determination of exacerbation risk of COVID-19 of a subject (hereinafter also referred to as "determination method"). In this method, S-IgM contained in a specimen collected from a subject is measured in the same manner as in the acquisition method of the present invention. Then, based on the value obtained by measurement of S-IgM, the exacerbation risk of COVID-19 of the subject is determined. For example, the measured value of S-IgM may be compared with a predetermined threshold value, and based on the comparison result, it may be determined whether the exacerbation risk of COVID-19 of the subject is high or low. Details of the predetermined threshold value corresponding to S-IgM are as described above.

In one embodiment, when the measured value of S-IgM is greater than or equal to the predetermined threshold value, it may be determined that the exacerbation risk of COVID-19 of the subject is high. In a further embodiment, when the measured value of S-IgM is less than the predetermined threshold value, it may be determined that the exacerbation risk of COVID-19 of the subject is low.

In a further embodiment, the determination method includes measuring S-IgM and IL-4 contained in a specimen collected from a subject, and the exacerbation risk of COVID-19 of the subject may be determined by comparing the measured values of S-IgM and IL-4 with predetermined threshold values corresponding thereto respectively. In one embodiment, when the measured value of S-IgM is greater than or equal to the first threshold value, or the measured value of IL-4 is greater than or equal to the second threshold value, it may be determined that the exacerbation risk of COVID-19 of the subject is high. In one embodiment, when the measured value of S-IgM is less than the first threshold value, and the measured value of IL-4 is less than the second threshold value, it may be determined that the exacerbation risk of COVID-19 of the subject is low.

In one embodiment, the determination method includes measuring S-IgM and IL-4 contained in a specimen collected from a subject, and the exacerbation risk of COVID-19 of the subject may be determined by the measured values of S-IgM and IL-4 as follows:
- when the measured value of S-IgM is greater than or equal to the first threshold value and the measured value of IL-4 is greater than or equal to the second threshold value, it is determined that the exacerbation risk of COVID-19 of the subject is high;
- when the measured value of S-IgM is greater than or equal to the first threshold value or the measured value of IL-4 is greater than or equal to the second threshold value, it is determined that the exacerbation risk of COVID-19 of the subject is moderate; and
- when the measured value of S-IgM is less than the first threshold value and the measured value of IL-4 is less than the second threshold value, it is determined that the exacerbation risk of COVID-19 of the subject is low.

In another embodiment, the exacerbation risk of COVID-19 of the subject may be determined by comparing the measured value of IL-4 with the second threshold value. In one embodiment, when the measured value of IL-4 is greater than or equal to the second threshold value, it may be determined that the exacerbation risk of COVID-19 of the subject is high. In one embodiment, when the measured value of IL-4 is less than the second threshold value, it may be determined that the exacerbation risk of COVID-19 of the subject is low.

In the present invention, a medical intervention for acute kidney injury or fibrosis of the lung can be performed on a subject determined to have a high exacerbation risk of COVID-19. The application discloses a method of treatment (hereinafter also referred to as "treatment method") of a COVID-19 patient having a high exacerbation risk and the invention provides therapeutic drugs and compositions capable of treating COVID-19 (such as drugs having antiviral action, drugs for reducing inflammation, and ACE inhibitors described above) for use in the methods of treatment disclosed herein. The treatment method disclosed herein includes measuring S-IgM contained in a specimen collected from a subject infected with SARS-CoV-2 or a subject suspected of suffering from COVID-19, determining the exacerbation risk of COVID-19 of the subject, based on a value obtained by the measurement of S-IgM, and performing medical intervention for severe COVID-19 on the subject determined to have a high risk. Details of the subject, the specimen, S-IgM and its measurement, the medical intervention and the like are the same as those described for the acquisition method of the present invention. For instance the step of performing medical intervention includes administering therapeutic drugs and compositions capable of treating COVID-19 as detailed herein.

Optionally, the treatment method includes measuring S-IgM and IL-4 contained in a specimen collected from a subject, and the exacerbation risk of COVID-19 of the subject may be determined by comparing the measured values of S-IgM and IL-4 with predetermined threshold values corresponding thereto respectively. Details of IL-4 and its measurement and determination are the same as those described for the acquisition method and the determination method of the present invention.

One related aspect of the invention provides a method for monitoring IgM antibody against S antigen of SARS-CoV-2 (hereinafter also referred to as "monitoring method"). In the monitoring method of the present invention, S-IgM contained in each specimen is measured using specimens collected from a subject at a plurality of time points. Details of the subject, the specimen, and S-IgM and its measurement are the same as those described for the acquisition method of the present invention.

In the monitoring methods of the present invention, the plurality of time points may be two or more different time points. For example, the plurality of time points includes a first time point and a second time point different from the first time point. The first time point is not particularly limited and can be any time point. For example, the first time point may be a time point when the subject is found to be infected with SARS-CoV-2, a time point when the subject develops symptoms of COVID-19, a time point when the subject is hospitalized, or the like The second time point is not particularly limited as long as it differs from the first time point. Preferably, the second time point is a time point when a period within one month has passed from the first time point. For example, the second time point is a time point when 0.5 hours, 1 hour, 2 hours, 3 hours, 4 hours, 5 hours, 6 hours, 7 hours, 8 hours, 9 hours, 10 hours, 12 hours, 15 hours, 18 hours, 1 day, 2 days, 3 days, 4 days, 5 days, 6 days, 7 days, 8 days, 9 days, 10 days, 12 days, 2 weeks, 3 weeks, 4 weeks or one month has passed from the first time point.

In the present invention, the "specimens collected from a subject at a plurality of time points" are specimens collected from the same subject at each of the plurality of time points. For example, the specimen collected from a subject at a plurality of time points includes a first specimen collected from a subject at a first time point and a second specimen collected from the subject at a second time point different from the first time point. In the monitoring method of the present invention, S-IgM may be measured each time a specimen is collected, or each collected specimen may be stored and measured collectively.

In the monitoring method of the present invention, the measured value of S-IgM in the same subject is monitored and serves as an index of exacerbation risk of COVID-19. By comparing the measured value of S-IgM of each specimen with a predetermined threshold value, the measured value of S-IgM may be used as an index of exacerbation risk of COVID-19 of a subject. Details of the predetermined threshold value are the same as those described for the acquisition method of the present invention.

In one embodiment of the monitoring methods of the invention, when the measured value of S-IgM is greater than or equal to the predetermined threshold value in at least one time point of the plurality of time points, it is suggested that the exacerbation risk of COVID-19 of the subject is high. In a further embodiment, when the measured value of S-IgM is less than the predetermined threshold value at all time points of the plurality of time points, it is suggested that the exacerbation risk of COVID-19 of the subject is low.

In a further embodiment, the determination method includes measuring S-IgM and IL-4 contained in each specimen collected from a subject at a plurality of time points, and the exacerbation risk of COVID-19 of the subject may be determined by comparing the measured values of S-IgM and IL-4 with predetermined threshold values corresponding thereto respectively. In one embodiment, when the measured value of S-IgM is greater than or equal to the first threshold value, or the measured value of IL-4 is greater than or equal to the second threshold value in at least one time point of the plurality of time points, it is suggested that the exacerbation risk of COVID-19 of the subject is high. In one embodiment, when the measured value of S-IgM is less than the first threshold value, and the measured value of IL-4 is less than the second threshold value at all time points of the plurality of time points, it is suggested that the exacerbation risk of COVID-19 of the subject is low.

In one embodiment, the monitoring method includes measuring S-IgM and IL-4 contained in a specimen collected from a subject, and classifies the exacerbation risk of COVID-19 of the subject into three stages by the measured values of S-IgM and IL-4. Specifically, the acquisition method is as follows:
- when the measured value of S-IgM is greater than or equal to the first threshold value, and the measured value of IL-4 is greater than or equal to the second threshold value in at least one time point of the plurality of time points, it is suggested that the exacerbation risk of COVID-19 of the subject is high;
- when the measured value of S-IgM is greater than or equal to the first threshold value, or the measured value of IL-4 is greater than or equal to the second threshold value in at least one time point of the plurality of time points, it is suggested that the exacerbation risk of COVID-19 of the subject is moderate; and
- when the measured value of S-IgM is less than the first threshold value, and the measured value of IL-4 is less than the second threshold value at all time points of the plurality of time points, it is suggested that the exacerbation risk of COVID-19 of the subject is low.

In another embodiment, by comparing the measured value of IL-4 with the second threshold value of each specimen collected at a plurality of time points, the measured value of IL-4 may be used as an index of exacerbation risk of COVID-19 of a subject. In one embodiment, when the measured value of IL-4 is greater than or equal to the second threshold value in at least one time point of the plurality of time points, it is suggested that the exacerbation risk of COVID-19 of the subject is high. In one embodiment, when the measured value of IL-4 is less than the second threshold value at all time points of the plurality of time points, it is suggested that the exacerbation risk of COVID-19 of the subject is low.

The conditions for terminating the monitoring method of the present invention are not particularly limited, and a healthcare professional such as a doctor may appropriately determine the conditions. For example, when the exacerbation risk of COVID-19 of the subject is suggested to be high by the measured values of S-IgM and/or IL-4 of the specimen collected from a subject at a plurality of time points, the monitoring method of the present invention may be terminated. In this case, it is preferable to perform medical intervention for severe COVID-19 on the subject. Details of the medical intervention are as described above. Alternatively, when the exacerbation risk of COVID-19 of the subject is suggested to be low by the measured values of S-IgM and/or IL-4 of the specimen collected from a subject at a plurality of time points, and symptoms of COVID-19 are not observed in the subject, the monitoring method of the present invention may be terminated.

In each of the above embodiments, when the measured values of S-IgM and IL-4 are the same as the threshold values corresponding thereto respectively, it has been suggested or determined that the exacerbation risk of COVID-19 of the subject is high, but it may be suggested or determined that the risk is low.

One further related aspect of the invention is a reagent kit for use in the acquisition method, the determination method or the monitoring method of the invention, or the treatment method described above. The reagent kit of the present invention includes a reagent containing a substance capable of specifically binding to S-IgM. In a further embodiment, the reagent kit may further include a reagent containing a substance capable of specifically binding to IL-4. In another embodiment, the reagent kit may further include a reagent containing a substance capable of specifically binding to IL-4, instead of the reagent containing a substance capable of specifically binding to S-IgM. The substances capable of specifically binding to each of S-IgM and IL-4 are as described above.

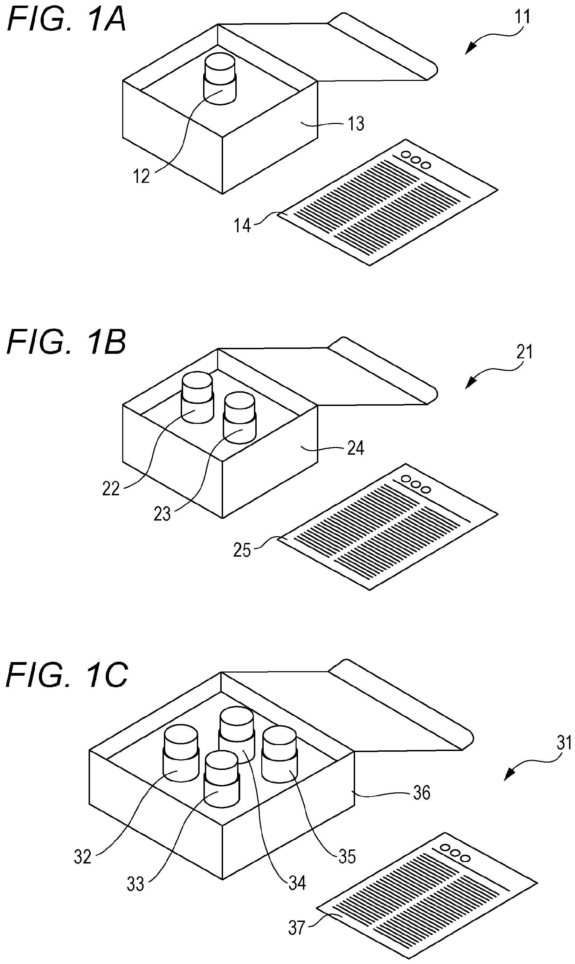

In the present invention, the reagent kit may be provided to a user by packing a container containing each reagent in a box. The box may contain an attached document. Configuration of the reagent kit, composition of each reagent, usage and the like may be described in the attached document. Fig. 1A shows an example of the reagent kit of the present invention. In Fig.1A, 11 denotes a reagent kit, 12 denotes a container containing a reagent containing a substance capable of specifically binding to S-IgM, 13 denotes a packing box, and 14 denotes an attached document.

In a further embodiment, the reagent kit may further include a reagent containing a substance capable of specifically binding to IL-4. In this case, reagent composition and usage of the reagent containing a substance capable of specifically binding to S-IgM and the reagent containing a substance capable of specifically binding to IL-4 and the like are described in the attached document. In another embodiment, a reagent containing a substance capable of specifically binding to IL-4 may be included, instead of the reagent containing a substance capable of specifically binding to S-IgM. In this case, reagent composition and usage of the reagent containing a substance capable of specifically binding to IL-4 and the like are described in the attached document.

In a preferred embodiment, the reagent kit of the present invention contains an S antigen for capturing S-IgM and S-IgM detection antibody. The S antigen may be immobilized on a solid phase, preferably a magnetic particle. Fig. 1B shows an example of the reagent kit of this embodiment. In Fig. 1B, 21 denotes a reagent kit, 22 denotes a first container containing a reagent containing an S antigen for capturing S-IgM, 23 denotes a second container containing a reagent containing a labeled antibody for S-IgM detection, 24 denotes a packing box, and 25 denotes an attached document.

In a further embodiment, the reagent kit may further include a reagent containing an IL-4 capture antibody and a reagent containing a labeled antibody for IL-4 detection. In another embodiment, a reagent containing an IL-4 capture antibody and a reagent containing a labeled antibody for IL-4 detection may be included, instead of the reagent containing S antigen and the reagent containing a labeled antibody for S-IgM detection.

It is preferable that any of the above reagent kits include a calibrator. Examples of the calibrator include a calibrator for quantification of S-IgM (S-IgM calibrator) and a calibrator for quantification of IL-4 (IL-4 calibrator). The S-IgM calibrator may include, for example, a buffer solution containing no S-IgM (negative control) and a SARS-CoV-2 IgM positive specimen (plasma or serum). The calibrator for IL-4 may include, for example, a buffer solution containing no IL-4 (negative control) and a buffer solution containing IL-4 at a known concentration.

Fig. 1C shows an example of a reagent kit including a calibrator. In Fig. 1C, 31 denotes a reagent kit, 32 denotes a first container containing a reagent containing S antigen, 33 denotes a second container containing a reagent containing a labeled antibody for S-IgM detection, 34 denotes a third container containing a buffer solution containing no S-IgM, 35 denotes a fourth container containing a SARS-CoV-2 IgM positive specimen, 36 denotes a packing box, and 37 denotes an attached document. The buffer solution containing no S-IgM and the SARS-CoV-2 IgM positive specimen can be used as a calibrator for S-IgM.

In a further embodiment, the reagent kit may further include a reagent containing an IL-4 capture antibody, a reagent containing a labeled antibody for IL-4 detection, and a calibrator for IL-4. In another embodiment, a reagent containing an IL-4 capture antibody, a reagent containing a labeled antibody for IL-4 detection and a calibrator for IL-4 may be included, instead of the reagent containing S antigen, the reagent containing a labeled antibody for S-IgM detection, and the calibrator for S-IgM.

The present invention also includes use of a reagent containing a substance capable of specifically binding to S-IgM for production of the reagent kit described above. One embodiment is use of a reagent for production of a reagent kit for acquiring information on exacerbation risk of COVID-19, in which the reagent is a reagent containing a substance capable of specifically binding to S-IgM. A further embodiment is use of a reagent for production of a reagent kit for assisting determination of exacerbation risk of COVID-19, in which the reagent is a reagent containing a substance capable of specifically binding to S-IgM. A further embodiment is use of a reagent for production of a reagent kit for monitoring S-IgM, in which the reagent is a reagent containing a substance capable of specifically binding to S-IgM. In these embodiments, the reagent kit may further contain a reagent containing a substance capable of specifically binding to IL-4. Alternatively, the reagent kit may contain a reagent containing a substance capable of specifically binding to IL-4, instead of the reagent containing a substance capable of specifically binding to S-IgM.

One related aspect of the present invention is an apparatus for acquiring information on exacerbation risk of COVID-19. Another related aspect provides a computer program for acquiring information on exacerbation risk of COVID-19.

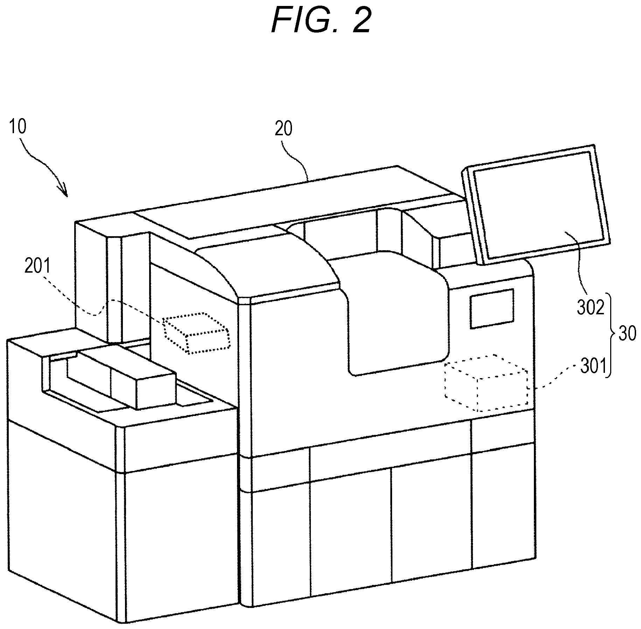

An example of an acquisition apparatus of the present invention will be described with reference to the drawings. However, the present invention is not limited only to the embodiment shown in this example. An acquisition apparatus 10 shown in Fig. 2 includes an immunoassay device 20 and a computer system 30.

The type of immunoassay device is not particularly limited, and it can be appropriately selected according to the method for measuring S-IgM and IL-4. When S-IgM and IL-4 are measured by ELISA, the immunoassay device is not particularly limited as long as it can detect a signal based on the used labeling substance. In the example shown in Fig. 2, the immunoassay device 20 is a commercially available automated immunoassay device capable of detecting a chemiluminescent signal generated by sandwich ELISA using a magnetic particle on which S antigen or anti-IL-4 antibody is immobilized and an enzyme-labeled S-IgM or IL-4 detection antibody.

The immunoassay device 20 includes a detection unit 201. When a specimen, a reagent containing the magnetic particle, and a reagent containing a detection antibody are set in the immunoassay device 20, the immunoassay device 20 performs an antigen-antibody reaction using each reagent. The immunoassay device 20 detects a chemiluminescent signal based on an enzyme-labeled antibody specifically bound to S-IgM or IL-4 by the detection unit 201. The immunoassay device 20 converts the detected chemiluminescent signal into a digital signal indicating the intensity thereof. The immunoassay device 20 transmits the obtained digital signal (hereinafter, referred to as "optical information") to computer system 30.

The computer system 30 includes a computer main body 301 and a display input unit 302. The computer system 30 receives the optical information from the immunoassay device 20. Then, a processor of the computer system 30 executes a computer program for acquiring information on exacerbation risk of COVID-19, installed in a solid state drive (hereinafter, referred to as "SSD") 313, based on the optical information. The display input unit 302 may be a touch panel in which an input unit is disposed on a surface of the display unit, and serves as both the display unit and the input unit. Examples of the touch panel include a touch panel of a known type such as a capacitance type. In the acquisition apparatus 10 shown in Fig. 2, the immunoassay device 20 and the computer system 30 are integrally configured, but the immunoassay device 20 and the computer system 30 may be separate devices.

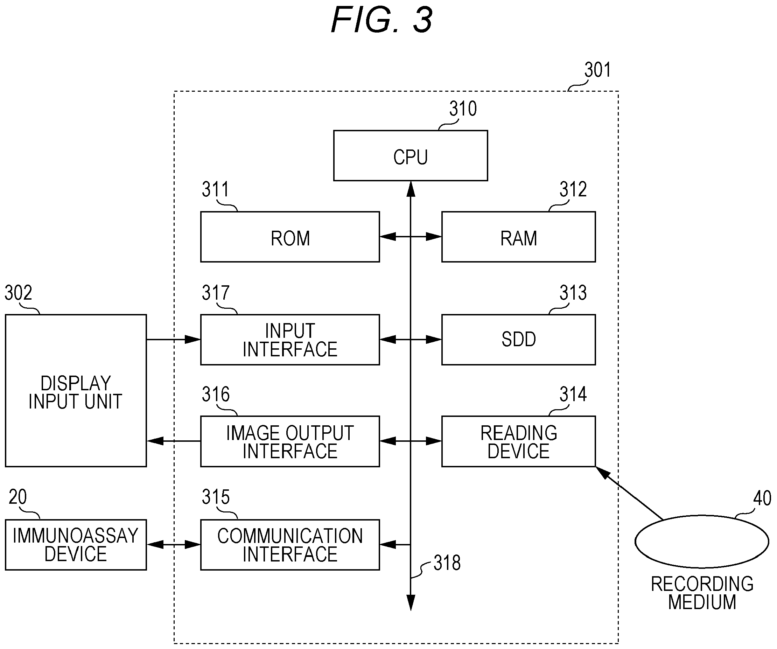

With reference to Fig. 3, a computer main body 301 includes a central processing unit (CPU) 310, a read only memory (ROM) 311, a random access memory (RAM) 312, an SSD 313, a reading device 314, a communication interface 315, an image output interface 316, and an input interface 317. The CPU 310, the ROM 311, the RAM 312, the SSD 313, the reading device 314, the communication interface 315, the image output interface 316 and the input interface 317 are data-communicably connected by a bus 318. Further, the immunoassay device 20 is communicably connected to the computer system 30 via the communication interface 315.

The CPU 310 can execute a program stored in the ROM 311 or the SSD 313 and a program loaded in the RAM 312. The CPU 310 calculates the measured value of S-IgM. The CPU 310 displays the measured value on the display input unit 302.

The ROM 311 may include mask ROM, PROM, EPROM or EEPROM. The ROM 311 stores a basic input output system (BIOS).

The RAM 312 may include SRAM or DRAM The RAM 312 is used for reading the program recorded in the ROM 311 and the SSD 313. The RAM 312 is also used as a work area of the CPU 310 when these programs are executed.

In the SSD 313, an operating system and a computer program such as an application program to be executed by the CPU 310, and data used for executing the computer program are installed. A hard disk drive may be used instead of the SSD. The application program includes a computer program for acquiring information on exacerbation risk of COVID-19. The data used for executing the computer program includes a threshold value for determining the exacerbation risk of COVID-19.

The reading device 314 is a device that can read a program or data recorded on a portable recording medium 40. The reading device 314 may include, for example, a flexible disk drive, a CD-ROM drive, a DVD-ROM drive, a USB port, an SD card reader, a CF card reader, or a memory stick reader.

The communication interface 315 is a wired interface conforming to a standard such as an Ethernet (registered trademark) interface. The computer main body 301 can also transmit print data to a printer or the like through the communication interface 315.

The image output interface 316 is an interface conforming to a predetermined standard. The predetermined standard may be D-Sub, DVI-I, DVI-D, HDMI (registered trademark), or DisplayPort. The image output interface 316 is connected to the display input unit 302 via a cable corresponding to the standard. As a result, the display input unit 302 can output a video signal corresponding to the image data coming from the CPU 310. The display input unit 302 displays an image (screen) according to the input video signal. The screen displayed on the display input unit 302 by the CPU 310 includes an element related to an operation such as an operation button.

The input interface 317 is an interface circuit that enables the CPU 310 to recognize a user's operation detected by the display input unit 302. The display input unit 302 detects a user's operation on an element such as a displayed operation button, and outputs a detection signal to the input interface 317. The input interface 317 drives the display input unit 302 so that the CPU 310 can recognize the detection signal from the display input unit 302 as, for example, the presence or absence of a touch, the position of the touch, and the like. The user can input various commands to the computer main body 301 through the display input unit 302.

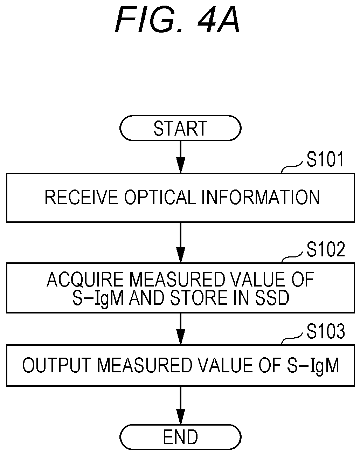

A processing procedure to be executed by the acquisition apparatus 10 of the present invention will be described with reference to the drawings. With reference to Fig. 4A, a processing procedure in the case of acquiring and outputting the measured value of S-IgM will be described. In this example, a measured value of S-IgM is acquired from a chemiluminescent signal generated by sandwich ELISA using a magnetic particle on which S antigen is immobilized and an enzyme-labeled S-IgM detection antibody and output. In another embodiment, the measured value of IL-4 may be acquired, instead of the measured value of S-IgM.

In step S101, the CPU 310 receives optical information from the immunoassay device 20. In step S102, the CPU 310 acquires the measured value of S-IgM from the received optical information. Specifically, the CPU 310 measures a calibrator containing S-IgM with a known concentration. The CPU 310 applies the optical information acquired in step S101 to a calibration curve prepared in advance. The CPU 310 converts the optical information into the concentration of S-IgM. The CPU 310 acquires the concentration as a measured value. The CPU 310 stores the measured value in the SSD 313. In step S103, the CPU 310 outputs the measured value of S-IgM. For example, the CPU 310 displays the measured value of S-IgM on the display input unit 302, the CPU 310 prints the measured value of S-IgM with a printer, or the CPU 310 transmits the measured value of S-IgM to a mobile device. When outputting the measured value of S-IgM, a predetermined threshold value corresponding to S-IgM may also be output as reference information. As described above, the acquisition apparatus of the present invention can provide the measured value of S-IgM to a doctor or the like as information on exacerbation risk of COVID-19. As described above, the measured value of S-IgM serves as an index of exacerbation risk of COVID-19.

In a further embodiment, measured values of S-IgM and IL-4 are acquired and output. For example, the CPU 310 acquires optical information (chemiluminescent signal) from the immunoassay device 20. The CPU 310 calculates measured values of S-IgM and IL-4 from the acquired optical information. The CPU 310 stores the calculated measured values in the SSD 313. The CPU 310 outputs the measured values of S-IgM and IL-4. For example, the CPU 310 displays the measured values of S-IgM and IL-4 on the display input unit 302, the CPU 310 prints the measured values with a printer, or the CPU 310 transmits the measured values to a mobile device. When outputting the measured values of S-IgM and IL-4, a first threshold value and a second threshold value may also be output as reference information.

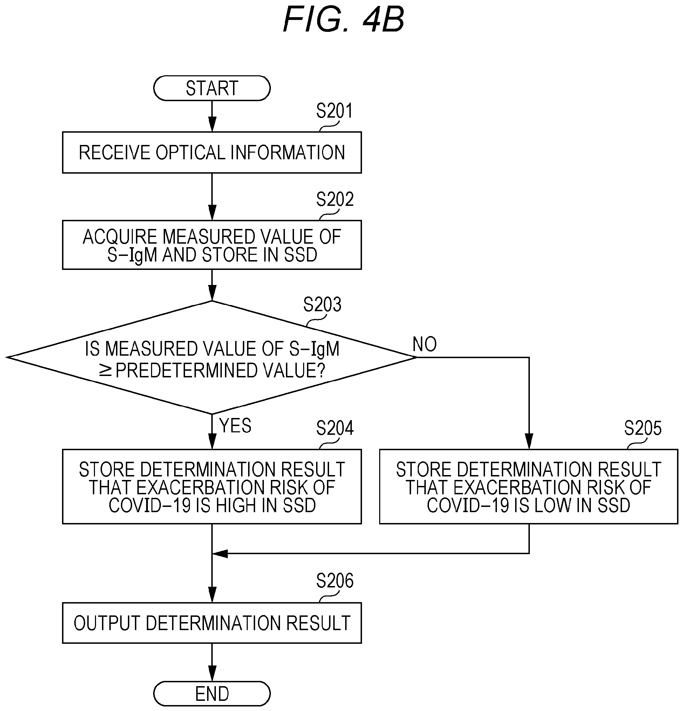

With reference to Fig. 4B, a flow in the case of determining the exacerbation risk of COVID-19 based on the measured value of S-IgM will be described. In step S201, the CPU 310 receives optical information from the immunoassay device 20. In step S202, the CPU 310 acquires the measured value of S-IgM from the received optical information by the same method as in step S102. The CPU 310 stores the measured value in the SSD 313. In step S203, the CPU 310 compares the acquired measured value of S-IgM with the predetermined threshold value stored in the SSD 313. When the measured value of S-IgM is greater than or equal to the predetermined threshold value, the process proceeds to step S204. In step S204, the CPU 310 stores a determination result that the exacerbation risk of COVID-19 is high in the SSD 313. In step S203, when the measured value of S-IgM is less than the threshold value, the process proceeds to step S205. In step S205, the CPU 310 stores a determination result that the exacerbation risk of COVID-19 is low in the SSD 313. In step S206, the CPU 310 outputs the determination result. For example, the CPU 310 displays the determination result on the display input unit 302, the CPU 310 prints the determination result with a printer, or the CPU 310 transmits the determination result to a mobile device. In this example, the measured value of IL-4 may be acquired, instead of the measured value of S-IgM. As described above, the acquisition apparatus of the present invention can provide the determination result of exacerbation risk of COVID-19 to a doctor or the like.

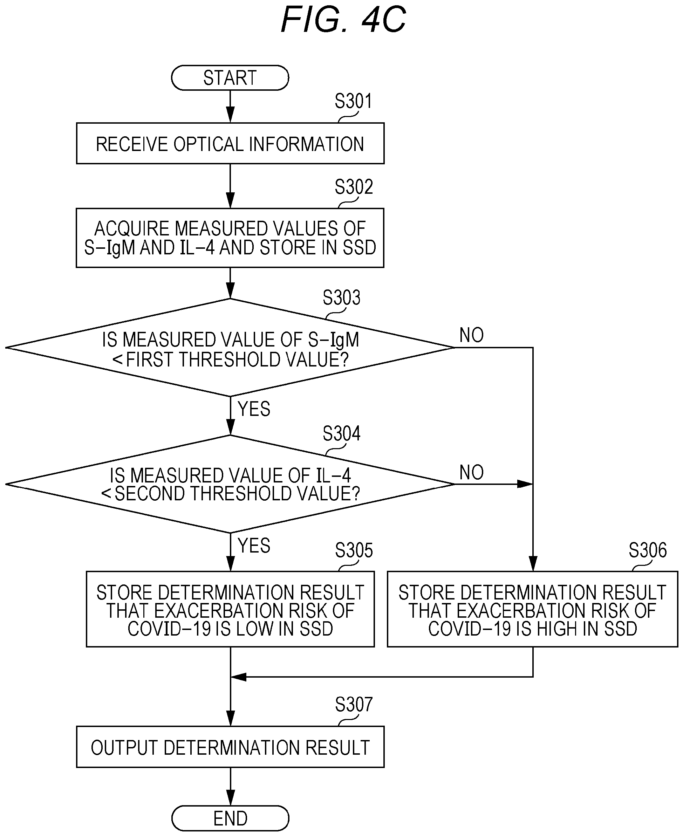

With reference to Fig. 4C, a flow in the case of determining the exacerbation risk of COVID-19 based on the measured values of S-IgM and IL-4 will be described. In step S301, the CPU 310 receives optical information from the immunoassay device 20. In step S302, the CPU 310 acquires the measured values of S-IgM and IL-4 from the received optical information by the same method as in step S102. The CPU 310 stores the measured value in the SSD 313. In step S303, the CPU 310 compares the acquired measured value of S-IgM with the first threshold value stored in the SSD 313. When the measured value of S-IgM is less than the first threshold value, the process proceeds to step S304. In step S304, the acquired measured value of IL-4 is compared with the second threshold value stored in the SSD 313. When the measured value of IL-4 is less than the second threshold value, the process proceeds to step S305. In step S305, the CPU 310 stores a determination result that the exacerbation risk of COVID-19 is low in the SSD 313.

In step S303, when the measured value of S-IgM is greater than or equal to the first threshold value, the process proceeds to step S306. In step S304, when the measured value of IL-4 is greater than or equal to the second threshold value, the process proceeds to step S306. In step S306, the CPU 310 stores a determination result that the exacerbation risk of COVID-19 is high in the SSD 313. In step S307, the CPU 310 outputs the determination result. For example, the CPU 310 displays the determination result on the display input unit 302, the CPU 310 prints the determination result with a printer, or the CPU 310 transmits the determination result to a mobile device. In this example, the order of processes of steps S303 and S304 can be changed.

In another embodiment, when the measured value of S-IgM is greater than or equal to the first threshold value and the measured value of IL-4 is greater than or equal to the second threshold value, the acquisition apparatus may determine that the exacerbation risk of COVID-19 is high. The flow in this case will be described. The CPU 310 receives optical information from the immunoassay device 20. The CPU 310 acquires the measured values of S-IgM and IL-4 from the received optical information by the same method as in step S102. The CPU 310 stores the measured values in the SSD 313. The CPU 310 compares the measured value of S-IgM with the first threshold value. When the measured value of S-IgM is greater than or equal to the first threshold value, the CPU 310 compares the measured value of IL-4 with the second threshold value. When the measured value of IL-4 is greater than or equal to the second threshold value, the CPU 310 stores a determination result that the exacerbation risk of COVID-19 is high in the SSD 313. When the measured value of S-IgM is less than the first threshold value or the measured value of IL-4 is lower than the second threshold value, the CPU 310 stores a determination result that the exacerbation risk of COVID-19 is low in the SSD 313. The CPU 310 outputs the determination result. For example, the CPU 310 displays the determination result on the display input unit 302, the CPU 310 prints the determination result with a printer, or the CPU 310 transmits the determination result to a mobile device.

Hereinbelow, the present invention will be described in detail by examples, but the present invention is not limited to these examples. Hereinafter, "HISCL" is a registered trademark of Sysmex Corporation.

### EXAMPLES

### Example 1

### (1) Specimen

Serum obtained from 24 patients whose SARS-CoV-2 infection was confirmed by PCR test was used as a specimen. The serum was prepared from blood collected on the day each patient was hospitalized. For the final medical condition of the patients after hospitalization, 5 of the 24 patients were rated "Critical" and 19 were rated "Severe". "Critical" is a case with severe pneumonia for which intensive care management including artificial respiration management is necessary or introduction of ECMO is considered, and "Severe" is a moderate case with pneumonia for which oxygen administration is necessary (SpO₂ ≤ 93%).

### (2) Measurement of biomarker in specimen

### (2.1) Measurement of antibody against SARS-CoV-2 antigen

As antibodies against SARS-CoV-2 antigen, serum concentrations of an IgG antibody and an IgM antibody against nucleocapsid protein (N antigen) of SARS-CoV-2 (hereinafter referred to as "N-IgG" and "N-IgM", respectively) and an IgG antibody and an IgM antibody against spike protein (S antigen) (hereinafter referred to as "S-IgG" and "S-IgM", respectively) were measured. The measurement was performed with a fully automated immunoassay device HISCL-5000 (Sysmex Corporation). For the measurement, the following reagents and the like were used.

### • Reagent containing magnetic particle on which SARS-CoV-2 antigen is immobilized (first reagent)

Based on genomic RNA sequences of SARS-CoV2 (NCBI accession numbers: YP_009724397 and YP_009724390), each of N antigen and S antigen (S1 subunit) of SARS-CoV-2 was prepared as follows. A His tag sequence was added to each sequence and cloned into a pcDNA 3.4 vector (Thermo Fisher Scientific), and the obtained expression vector was transfected into Expi293 cell (Thermo Fisher Scientific). After 6 days, culture supernatant was collected. Recombinant antigens in the culture supernatant were purified using HisTrap HP column (Cytiva) and HiLoad 26/600 Superdex 200 pg column (Cytiva). Each of the purified recombinant N antigen and S antigen was immobilized on the surface of a magnetic particle using 1-ethyl-3-(3-dimethylaminopropyl) carbodiimide (Dojindo Molecular Technologies Inc.) and N-hydroxysuccinimide (Sigma-Aldrich). The magnetic particle on which each antigen was immobilized was washed 3 times with a 10 mM HEPES buffer solution (pH 7.5). The washed magnetic particle was added to 10 mM HEPES (pH 7.5) so that the concentration of magnetic particle was 0.48 to 0.52 mg/mL to obtain a reagent containing a magnetic particle on which the recombinant N antigen was immobilized and a reagent containing a magnetic particle on which the recombinant S antigen was immobilized.

### • Reagent containing ALP-labeled antibody against human IgG or human IgM (second reagent)

An antibody that specifically binds to human IgG was labeled with ALP by a common method and dissolved in a buffer containing 1% BSA and 0.5% casein. The same applies to an antibody that specifically binds to human IgM.

### • Measurement buffer and ALP substrate solution

As a measurement buffer, a HISCL R4 reagent (Sysmex Corporation) was used. HISCL R5 reagent (Sysmex Corporation) containing CDP-Star (registered trademark) (Applied Biosystems) was used as a solution of chemiluminescent substrate of ALP.

A measurement procedure according to HISCL-5000 was as follows. Serum (20 µL) and the first reagent (50 µL) were mixed. The magnetic particle in the obtained mixed solution was magnetically collected to remove the supernatant, and a HISCL washing solution (300 µL) was added to wash the magnetic particle. The supernatant was removed, and the second reagent (100 µL) was added to the magnetic particle and mixed. The magnetic particle in the obtained mixed solution was magnetically collected to remove the supernatant, and a HISCL washing solution (300 µL) was added to wash the magnetic particle. The supernatant was removed, and the measurement buffer (50 µL) and the ALP substrate solution (100 µL) were added to the magnetic particle, and the chemiluminescence intensity was measured. As a calibrator, a SARS-CoV-2 positive specimen (Cantor Bioconnect and TRINA BIOREACTIVES AG) was serially diluted with a phosphate buffer and used. The calibrator was measured 3 times, and a calibration curve was prepared by logistics regression analysis. The chemiluminescence intensity obtained by the measurement of each serum was applied to the calibration curve to determine the concentration of antibody.

### (2.2) Measurement of IL-4

The concentration of IL-4 in the serum was measured using Human IL-4 SimpleStep ELISA kit (ab215089, Abcam). A diluted standard sample (50 µL) and the serum (50 µL) of each patient were added to each well of a plate. Next, an antibody cocktail (50 µL) was added to each well. The plate was sealed and incubated on a plate shaker set at 400 rpm at room temperature for 1 hour. The plate was washed 3 times with 1 × Wash Buffer PT. In each washing step, Wash Buffer PT was added, and the mixture was allowed to stand for 30 seconds. Next, TMB Development Solution (100 µL) was added to each well, and the mixture was incubated on a plate shaker in the dark for 10 minutes. Stop Solution (100 µL) was then added to each well and mixed on a plate shaker for 1 minute. OD at 450 nm was measured with a Vmax microplate reader (Molecular Devices, LLC). The concentration of IL-4 in the serum of each patient was calculated based on the standard curve prepared from the measured value of the standard sample.

### (3) Measurement results

The measurement results of the antibodies are shown in Table 1. In the table, the value of antibody concentration is a median value, the value in parentheses indicates distribution range, and "n.s." indicates that the difference was not significant. The measurement result of IL-4 is shown in Table 2. In the table, the value of IL-4 concentration is a median value, and the value in parentheses indicates distribution range.

**[Table 1]**

| Antibody concentration in serum during hospitalization (AU/mL) | | | |
|---|---|---|---|
| Antibody | Severe | Critical | P Value |
| N-IgG | 6.6 (1.6-78.8) | 51.5 (2.1-156.4) | n.s |
| S-IgG | 0.5 (0.3-2.3) | 6.3 (1.1-104.5) | n.s |
| N-IgM | 7.6 (3.5-59.5) | 15.2 (9.4-28.4) | n.s |
| S-IgM | 9.4 (3.5-37.5) | 125.8 (41.7-304.5) | p<0.05 |

**[Table 2]**

| IL-4 concentration in serum during hospitalization (pg/mL) | | | |
|---|---|---|---|
| | Severe | Critical | P Value |
| IL-4 | 0 (0-10.84) | 1.34 (0-5.01) | p<0.05 |

As shown in Table 1, the concentrations of four antibodies against SARS-CoV-2 antigen tended to be higher in the critical group than in the severe group. However, an antibody in which a significant difference was observed between the critical group and the severe group was only S-IgM. This result suggested that S-IgM can be used as a biomarker for determining the exacerbation risk of COVID-19. As shown in Table 2, the IL-4 concentration was significantly higher in the critical group than in the severe group. IL-4 is considered to be important for class switching from IgM to IgG, and the measurement result of IL-4 in the critical group is considered to be related to the measurement result of S-IgM in the same group. In any case, it was suggested that IL-4 can also be used as a biomarker for determining the exacerbation risk of COVID-19.

## Claims

1. A method for assisting determination of exacerbation risk of COVID-19, comprising:
measuring IgM antibody against S antigen of SARS-CoV-2 contained in a specimen collected from a subject infected with SARS-CoV-2 or a subject suspected of suffering from COVID-19; and
determining the exacerbation risk of COVID-19 of the subject, based on a value obtained by the measurement of IgM antibody.

2. The method according to claim 1, wherein when the value is greater than or equal to a predetermined threshold value, it is determined that the exacerbation risk of COVID-19 of the subject is high.

3. The method according to claim 1 or 2, wherein when the value is less than the predetermined threshold value, it is determined that the exacerbation risk of COVID-19 of the subject is low.

4. The method according to any one of claims 1 to 3, wherein the specimen is whole blood, plasma or serum.

5. A method for acquiring information on exacerbation risk of COVID-19, comprising measuring IgM antibody against S antigen of SARS-CoV-2 contained in a specimen collected from a subject infected with SARS-CoV-2 or a subject suspected of suffering from COVID-19, wherein a value obtained by the measurement of IgM antibody serves as an index of exacerbation risk of COVID-19 of the subject.

6. The method according to claim 5, wherein when the value is greater than or equal to a predetermined threshold value, it is suggested that the exacerbation risk of COVID-19 of the subject is high.

7. The method according to claim 5 or 6, wherein when the value is less than the predetermined threshold value, it is suggested that the exacerbation risk of COVID-19 of the subject is low.

8. A method for monitoring IgM antibody against S antigen of SARS-CoV-2, using a first specimen collected at a first time point from a subject infected with SARS-CoV-2 or a subject suspected of suffering from COVID-19 and a second specimen collected at a second time point from the subject, comprising measuring IgM antibody against S antigen of SARS-CoV-2 contained in each of the first and second specimens, wherein values obtained by the measurements of IgM antibody serve as indices of exacerbation risk of COVID-19 of the subject.

9. The method according to claim 8, wherein when at least one of the value is greater than or equal to a predetermined threshold value, it is suggested that the exacerbation risk of COVID-19 of the subject is high.

10. The method according to claim 9, wherein when all of the values are less than the predetermined threshold value, it is suggested that the exacerbation risk of COVID-19 of the subject is low.

11. Use of a reagent kit in the method according to any one of claims 1 to 10, the reagent kit comprising a reagent comprising a substance capable of specifically binding to IgM antibody against S antigen of SARS-CoV-2.

12. An apparatus for acquiring information on exacerbation risk of COVID-19, comprising:
a computer comprising a processor and a memory under control of the processor, wherein the memory is recorded with a computer program for executing on the computer the following steps of:
acquiring a value obtained by measurement of IgM antibody against S antigen of SARS-CoV-2 contained in a specimen collected from a subject infected with SARS-CoV-2 or a subject suspected of suffering from COVID-19; and
outputting the value, and
wherein the value serves as an index of exacerbation risk of COVID-19 of the subj ect.

13. The apparatus according to claim 12, wherein the computer program further executes on the computer a step of determining the exacerbation risk of COVID-19 of the subject, based on the measured value of IgM antibody.

14. The apparatus according to claim 13, wherein when the value is greater than or equal to a predetermined threshold value, it is determined that the exacerbation risk of COVID-19 of the subject is high.

15. The apparatus according to claim 14, wherein when the value is less than the predetermined threshold value, it is determined that the exacerbation risk of COVID-19 of the subject is low.
